# EUROPEAN PATENT APPLICATION

(11) **EP 4 772 025 A1**
(43) Date of publication of application: **08.07.2026**
(21) Application number: 24859911.0
(22) Date of filing: 29.08.2024
(51) Int. Cl.: A23L 33/135, A23C 9/00, A23C 9/127, A23L 2/52, A23L 33/10, A23L 33/125, A61K 35/745, A61P 1/00, A61P 1/04, A61P 1/10, A61P 1/12, A61P 1/14, A61P 25/20, A61P 25/22, A61P 25/24, C12N 1/20, C12N 15/09

(54) **BACTERIUM, COMPOSITION, AND METHOD FOR PRODUCING COMPOSITION**

(30) Priority: 31.08.2023 JP 2023141728; 31.08.2023 JP 2023141717
(71) Applicant: Morinaga Milk Industry Co., Ltd., Minato-ku Tokyo 105-7122 (JP)
(72) Inventor: MUTO, Masamichi, Zama-shi, Kanagawa 252-8583 (JP); NOMURA, Naoko, Zama-shi, Kanagawa 252-8583 (JP); SATO, Soichiro, Zama-shi, Kanagawa 252-8583 (JP); XU, Chendong, Zama-shi, Kanagawa 252-8583 (JP); AOKI, Takahiro, Zama-shi, Kanagawa 252-8583 (JP); MIYAMOTO, Shu, Zama-shi, Kanagawa 252-8583 (JP)
(74) Representative: Vossius & Partner Patentanwälte Rechtsanwälte mbB
(86) International application number: PCT/JP2024/030991
(87) International publication number: WO 2025/047884

(57) **Abstract**

The bacterium is *Bifidobacterium bifidum* MCC2085 (NITE BP-03881). A composition contains the bacterium. The composition is a food or drink composition. The food or drink composition is a probiotic composition or a nutritional composition. A method for producing a composition includes a step of mixing the bacterium and raw material other than the bacterium.

## Description

### Technical Field

The present invention relates to a bacterium, a composition and a method for producing a composition.

The present application claims priority from patent application No. 2023-141728 and patent application No. 2023-141717 which were filed in Japan on August 31, 2023, and the contents of both applications are incorporated here by reference.

### Background Art

In recent years, many studies for the purpose of inhibiting onset of diseases or promoting health or another purpose by actively taking bacteria which positively influence animals (so-called good bacteria, also called "probiotics") and regulating the gut environment have been conducted.

For example, PTL 1 discloses probiotics which have an immunomodulatory effect and which are useful for treating a lesion related to a change in the immune system.

Moreover, studies for inhibiting onset of diseases or promoting health by improving the intestinal microbiota including good bacteria have also been conducted. It is known that a major example of improvement of the intestinal microbiota is promotion of proliferation of good bacteria and that the promotion has beneficial effects on human health such as an intestinal regulation and preventing and improving inflammatory bowel diseases (NPLs 1 to 3).

### Citation List

### Patent Literature

PTL 1: JP2013-521335A

### Non Patent Literature

NPL 1: Cannarella et al., "Mixture of probiotics reduces inflammatory biomarkers and improves the oxidative/nitrosative profile in people with rheumatoid arthritis", Nutrition (Burbank, Los Angeles County, Calif.), vol. 89, 2021

NPL 2: Kim et al., "Probiotic Supplementation Improves Cognitive Function and Mood with Changes in Gut Microbiota in Community-Dwelling Older Adults: A Randomized, Double-Blind, Placebo-Controlled, Multicenter Trial", Journals of Gerontology: Biological Sciences, vol. 76, No. 1, 2021

NPL 3: Gomi et al., "Bifidobacterium bifidum YIT 10347 fermented milk exerts beneficial effects on gastrointestinal discomfort and symptoms in healthy adults: A double-blind, randomized, placebo-controlled study", Journal of Dairy Science, vol. 101, No. 6, 2018

### Summary of Invention

### Technical Problem

Thus, further studies and development of probiotics are required.

The invention provides a novel bacterium belonging to *Bifidobacterium bifidum,* a composition containing the bacterium and a method for producing the composition.

### Solution to Problem

The invention includes the following aspects.
[1] A bacterium which is *Bifidobacterium bifidum* MCC2085 (NITE BP-03881).
[2] A composition containing the bacterium according to [1].
[3] The composition according to [2] which is a food or drink composition.
[4] The composition according to [3] in which the food or drink composition is a probiotic composition or a nutritional composition.
[5] The composition according to [4] in which the nutritional composition is a formula.
[6] The composition according to [4] or [5] further containing a human milk oligosaccharide.
[7] The composition according to [2] which is a pharmaceutical composition.
[8] A method for producing a composition including a step of mixing the bacterium according to [1] and raw material.

As another aspect, the invention includes the following aspects.
[1] A food or drink composition containing a bacterium which is *Bifidobacterium bifidum* MCC2085 (NITE BP-03881) and a raw food material,
   in which the bacterium is viable bacterial cells, and
   the bacterium content of the composition is 1×10³ to 1×10¹² CFU/g.
[2] A method for producing a food or drink composition containing a bacterium which is *Bifidobacterium bifidum* MCC2085 (NITE BP-03881), including
   a step of mixing at least one of the bacterium and a culture of the bacterium, and a raw food material,
   in which the mixing step is a step of mixing in such a manner that the bacterium content of the food or drink composition becomes 1×10³ to 1×10¹² CFU/g.
[3] A method for improving intestinal microbiota including administering a bacterium which is *Bifidobacterium bifidum* MCC2085 (NITE BP-03881) to a subject of administration at an intake of 1×10³ to 1×10¹² CFU/day.
[4] Use of a bacterium which is *Bifidobacterium bifidum* MCC2085 (NITE BP-03881) for the improvement of intestinal microbiota.
[5] An oral composition for reaching an intestine containing *Bifidobacterium bifidum* MCC2085 (NITE BP-03881).

### Advantageous Effects of Invention

According to the aspects, a novel bacterium of *Bifidobacterium* which acts as a probiotic, a composition containing the bacterium and a method for producing the composition can be provided.

The invention provides novel *Bifidobacterium bifidum* MCC2085 (accession number NITE BP-03881, the survival rate is 79% when cultured for 120 minutes in the presence of artificial gastric juice having pH 3.0), which has superior acid resistance to that of *Bifidobacterium bifidum* ATCC29521 (the survival rate is 3.1% when cultured for 120 minutes in the presence of artificial gastric juice having pH 3.0). Furthermore, the production amount of novel *Bifidobacterium bifidum* MCC2085 (NITE BP-03881) increased to 3.2 times the amount of the control, and the production amount of ATCC29521 increased to 1.7 times the amount of the control. Thus, MCC2085 had a superior serotonin-producing ability to that of ATCC29521.

### Brief Description of Drawings

[FIG. 1] FIG. 1 is a graph showing the changes with time of the bacterial cell concentrations (CFU/ml) of MCC2085 and ATCC29521 in Test Example 2.
[FIG. 2] FIG. 2 is a graph showing the changes with time of the survival rates (%) of MCC2085 and ATCC29521 in Test Example 2.
[FIG. 3] FIG. 3 is a graph showing the amounts of serotonin produced by MCC2085 and ATCC29521.

### Description of Embodiments

A preferable embodiment for carrying out the invention is explained below. In this regard, the embodiment explained below shows an example of typical embodiments of the present disclosure, and the scope of the present technique is not construed as being narrower by the embodiment. In the present specification, a numerical range expressed with "a lower limit to an upper limit" means that the upper limit and the lower limit are included.

*Bifidobacterium* spp. have been reported to have various physiological functions. It has been reported that proliferation in the intestines or the substances produced by *Bifidobacterium* spp. (for example, acetic acid, etc.) are involved in the physiological functions. Accordingly, the bacterium of *Bifidobacterium* of the embodiment can also be expected to be highly safe and show the generally known efficacies of *Bifidobacterium* spp. in a wide age group. Therefore, the bacterium of *Bifidobacterium* of the embodiment can be used for a wide range of compositions, such as a food or a drink, a functional food use, a pharmaceutical use and a feed use. Moreover, the bacterium of *Bifidobacterium* of the embodiment can be expected to have a probiotic effect and thus can also be used for the purpose of promoting the health, improving the diet, improving the gut environment (intestinal regulation) or the like.

### <Bifidobacterium bifidum MCC2085 (NITE BP-03881)>

*Bifidobacterium bifidum* MCC2085 (NITE BP-03881, also simply referred to as "MCC2085" below) is novel *Bifidobacterium bifidum* isolated from feces of a human infant as shown in Test Example 1 below and was deposited for an international deposit as a novel bacterium on April 13, 2023 to NITE Patent Microorganisms Depositary (NPMD), National Institute of Technology and Evaluation (address: #122, 2-5-8 Kazusakamatari, Kisarazu-shi, Chiba 292-0818, Japan) as *Bifidobacterium bifidum* MCC2085 (accession number: NITE BP-03881). The bacterium can be generally acquired from the preservation organization.

MCC2085 has a property of having a high survival rate in an environment with low pH, namely high acid resistance. Moreover, when an animal takes MCC2085, the intestinal regulation effect is expected to be obtained. In the present specification, the pH means a value measured at 25°C.

In the present specification, the "high acid resistance" can be evaluated by calculating the survival rate in Test Example 2 below. MCC2085 is inoculated in 1 mL of artificial gastric juice (pH 3.0) at 1 v/v%, and thus a mixture is obtained. The obtained mixture is maintained at 37°C. The point in time at which the container containing the mixture is set in the incubator is regarded as 0 minute. Samples are taken from the mixture at 0 minute and after 120 minutes and cultured by pour culture for 72 hours, and the CFUs (Colony Forming Units) are measured. When the ratio (%) of the CFU of the pour culture after 120 minutes to the CFU of the pour culture at 0 minute is 70% or more, more preferably 75% or more, it is determined that the bacterium "has a high survival rate in an environment with low pH", namely "has high acid resistance".

MCC2085 is not limited to the bacterium itself deposited and registered at the organization under the bacterial name (also called "deposited bacterium" below for the convenience of explanation), but includes a substantially equivalent bacterium thereof (also called "derived strain" or "induced strain"). Regarding the bacterium, a "substantially equivalent bacterium of the deposited bacterium" means a bacterium which belongs to the identical species to that of the deposited bacterium and which has equivalent or higher acid resistance to that of the deposited bacterium of this case. The substantially equivalent bacterium of the deposited bacterium may be, for example, a derived strain of the deposited bacterium. The derived strain is a bacterium bred from the deposited bacterium or a bacterium naturally generated from the deposited bacterium.

In the embodiment, the substantially equivalent bacterium of MCC2085 is, for example, a bacterium of the same genus in which the base sequence of 16S rRNA gene has a homology of preferably 99.9% or more, more preferably 100% to the base sequence of 16S rRNA gene of MCC2085 and which preferably has the identical bacteriological properties to those of MCC2085.

In the embodiment, MCC2085 includes a mutant of MCC2085 as long as the mutant has the features of MCC2085 (for example, a high survival rate in an environment with low pH, and a high serotonin production amount). Moreover, the mutant is preferably a bacterium which has the identical bacteriological properties to those of MCC2085 and which has an equivalent or higher survival rate in an environment with low pH than that of MCC2085. Whether a mutant has "an equivalent or higher survival rate in an environment with low pH" than that of MCC2085 can be examined, for example, by the method of Test Example 2 described below.

Such a mutant may be constructed by introduction of a non-artificial mutation to MCC2085. Moreover, such a mutant may be constructed by introduction of a mutation to the bacterium through treatment using a mutagen such as UV or constructed by introduction of a mutation to the bacterium through various genetic engineering methods.

Examples of the substantially equivalent bacterium and the derived strain include the bacteria below.
(1) A bacterium which is identified as the identical bacterium by the Randomly Amplified Polymorphic DNA method or the Pulsed-field gel electrophoresis method (described in Probiotics in food/Health and nutritional properties and guidelines for evaluation 85 Page 43)
(2) A bacterium which possesses the deposited bacterium-derived genes only and does not have any exogenous genes and which has a DNA identity of 95% or more (preferably 98% or more)
(3) A bacterium having the identical characteristics to those of a bacterium bred from the bacterium (including genetic engineering modification, mutation or spontaneous mutation)

MCC2085 can be proliferated by culturing the bacterium. The culture method is not particularly limited as long as MCC2085 can be proliferated, and a method which is generally used for culturing *Bifidobacterium* spp. can be used with appropriate modification as necessary. For example, the culture temperature may be 30 to 50°C and is preferably 35 to 45°C. MCC2085 is preferably cultured under an anaerobic condition and can be cultured, for example, while sending an anaerobic gas such as carbon dioxide gas. Furthermore, MCC2085 may be cultured under a microaerophilic condition such as static liquid culture.

The culture medium for culturing MCC2085 for proliferation is not particularly limited, and a culture medium which is generally used for culturing *Bifidobacterium* spp. can be used with appropriate modification as necessary. For example, as a carbon source, saccharides such as galactose, glucose, fructose, mannose, cellobiose, maltose, lactose, sucrose, trehalose, starch hydrolysate and molasses can be used. As a nitrogen source, for example, ammonia and ammonium salts and nitrates such as ammonium chloride and ammonium nitrate can be used. Moreover, as an inorganic salt, for example, sodium chloride, potassium chloride, potassium sulfate, magnesium sulfate, calcium chloride, calcium nitrate, manganese chloride, and ferrous sulfate can be used. Furthermore, organic components such as soybean powder, a defatted soybean cake, meat extract and yeast extract may also be used. As a prepared culture medium, for example, MRS culture medium can be preferably used.

As MCC2085, the obtained culture can be used directly after culture or can be diluted or concentrated, or bacterial cells only can be collected from the culture. As long as the effects of the invention are exhibited, bacterial cells of MCC2085 which have been subjected to additional treatment such as heating, drying, disruption and sterilization after culture can also be used. The treatments can be used appropriately in combination.

The drying method may be a spray drying method or a lyophilization method. The sterilization method may be a retort sterilization method, a UHT (Ultra High Temperature) sterilization method, a pressurized sterilization method, a high-pressure steam sterilization method, a dry heat sterilization method, a flow steam sterilization method, an electromagnetic wave sterilization method, an electron beam sterilization method, a high-frequency sterilization method, a radiation sterilization method, a ultraviolet sterilization method, an ethylene oxide gas sterilization method, a hydrogen peroxide gas plasma sterilization method, a chemical sterilization method (specifically, an alcohol sterilization method, a formalin fixation method, and an electrolyzed water treatment method) or the like.

MCC2085 can be, for example, in the form of a bacterial cell concentrate, lyophilized bacterial cells, spray dried bacterial cells, sterilized cells, heat sterilized cells, or a disrupted bacterial cell.

### <Composition Containing MCC2085>

The composition of the embodiment contains MCC2085. The composition includes a food or drink composition, a pharmaceutical composition and the like. MCC2085 is derived from a human and thus has few side effects and is highly safe, and thus an animal can take MCC2085 continuously for a long time. Accordingly, MCC2085 can be used for products for a wide variety of uses such as a food or drink composition and a pharmaceutical composition.

When an animal takes the composition of the embodiment, the intestinal regulation effect is expected to be obtained. Viable bacterial cells or sterilized cells of MCC2085 can be used after mixing with a general carrier or diluent or the like which is acceptable physiologically or pharmaceutically or in terms of foods and drinks.

The MCC2085 content of the entire composition of the embodiment is not particularly limited, and may be, for example, 10³ CFU/g or mL or more, 10⁴ CFU/g or mL or more, 10⁵ CFU/g or mL or more, or 10⁶ CFU/g or mL or more, and may be 10¹² CFU/g or mL or less, 10¹¹ CFU/g or mL or less, or 10¹⁰ CFU/g or mL or less.

The MCC2085 content of the composition of the invention may be 10³ CFU/g or mL to 10¹² CFU/g or mL, 10⁹ CFU/g or mL to 10¹¹ CFU/g or mL, or 10⁵ CFU/g or mL or more to 10¹⁰ CFU/g or mL.

The composition containing MCC2085 may be for use for a therapeutic purpose or use for a non-therapeutic purpose.

The "non-therapeutic purpose" is a concept which does not include medical practice, namely treatment of a human body by therapy. Examples thereof include health promotion and beauty treatment.

The "improvement" means: improvement of a disease, a symptom, or the condition; prevention or delay of deterioration of a disease, a symptom, or the condition; or reversal, prevention, or delay of progress of a disease or a symptom.

The "prevention" means prevention or delay of the onset of a disease or a symptom in the subject of the application or reduction of the risk of a disease or a symptom of the subject of the application.

When the composition of the invention is used for a non-therapeutic purpose, the composition can be administered to a healthy individual as the subject. The healthy individual may mean an individual having healthy intestinal microbiota or an individual without any disease caused by a disorder of the intestinal microbiota. Examples of the disease caused by a disorder of the intestinal microbiota include diarrhea and constipation. When the composition of the invention is used for a non-therapeutic purpose, the composition is used for regulating the excellent intestinal microbiota of a healthy individual further better, and can be used, in particular, for proliferating MCC2085 in the intestines of a healthy individual.

When the composition of the invention is used for a non-therapeutic purpose, the composition can regulate the intestinal microbiota of a healthy individual and can prevent diarrhea and/or constipation.

When the composition of the invention is used for a therapeutic purpose, the composition can be administered to a non-healthy individual as the subject. An example of the non-healthy individual is an individual having a disease caused by a disorder of the intestinal microbiota.

When the composition of the invention is used for a therapeutic purpose, the composition can regulate the intestines of a non-healthy individual, and improvement, prevention or treatment of diarrhea or constipation can become possible.

When the composition of the invention is used for a therapeutic purpose, the composition is used for improving the intestinal microbiota of a non-healthy individual and can be used, in particular, for proliferating MCC2085 in the intestines of a non-healthy individual.

The composition containing MCC2085 may be used for a human or a nonhuman animal (preferably a mammal) as the subject of application, preferably for a human or a pet, more preferably for a human. Furthermore, the subject to which the present technique is applied is not particularly limited as long as the subject wishes for a probiotic effect, and examples thereof include an infant, a small child, a child, an adult, a middle-aged individual, an aged individual, a healthy individual, and those with poor gut environment. Of these, the present technique is preferably used for an infant, a small child, an adult, an aged individual or those with poor enteric environment.

Regarding the administration or the intake of MCC2085, MCC2085 is preferably taken continuously for at least one week, or more preferably taken continuously for at least four weeks. During the administration or intake period of MCC2085, MCC2085 is desirably taken every day.

The used amount of MCC2085 is not particularly restricted because of the high safety, and for example, the amount is preferably 1×10⁵ to 1×10¹² CFU/kg body weight/day, more preferably 1×10⁷ to 1×10¹¹ CFU/kg body weight/day, further preferably 1×10⁸ to 1×10¹⁰ CFU/kg body weight/day. Alternatively, the used amount per one individual (body weight) (in other words, dose) is preferably 10⁷ to 10¹⁴ CFU/day, more preferably 10⁸ to 10¹³ CFU/day, further preferably 10⁹ to 10¹² CFU/day. When MCC2085 is sterilized cells, the CFU can be replaced with the cells.

Moreover, the used amount of MCC2085 is not particularly restricted because of the high safety, and the dry weight of the bacterial cells is preferably 0.01 to 10000 mg/body weight kg/day, more preferably 0.01 to 1000 mg/body weight kg/day, still further preferably 0.1 to 100 mg/body weight kg/day.

The used amount of MCC2085 is not particularly restricted because of the high safety, and is preferably 0.001 to 100 mL/body weight kg/day, more preferably 0.1 to 10 mL/body weight kg/day.

### <Pharmaceutical Composition>

The pharmaceutical composition of the embodiment is not particularly restricted as long as the pharmaceutical composition contains MCC2085. The pharmaceutical composition can also be used as an intestinal regulation composition containing MCC2085 or the like.

The use of the pharmaceutical composition of the embodiment is for a non-healthy individual as a subject, and the pharmaceutical composition is used for the therapeutic purpose above.

The dosage form of the pharmaceutical composition of the embodiment is not particularly restricted. Specifically, examples of the dosage form of the pharmaceutical composition include tablets, pills, powder, liquid preparation, suspensions, emulsions, granules, capsules, syrups, suppositories, injections, ointment, patches, eye drops, and nasal drops. Moreover, for the formulation, an additive which is generally used as a carrier for formulation, such as excipients, binders, disintegrating agents, lubricants, stabilizers, flavoring agents, diluents, surfactants and solvents for injection, can be used.

For the formulation, a component which is generally used for formulation, such as excipients, pH-adjusting agents, colorants and corrigents, can be used for the pharmaceutical composition according to the embodiment. As long as the effects of the invention are not impaired, a component having an effect of preventing, improving and/or treating a disease or a symptom which is known or will be found in the future and which is relevant to the embodiment can also be used for the pharmaceutical composition according to the embodiment. In addition, the formulation can be appropriately conducted by a known method depending on the dosage form. The formulation may also be conducted by appropriately blending a carrier for formulation.

The MCC2085 content of the pharmaceutical composition of the embodiment is appropriately determined based on the dosage form, the usage, the age of the patient, the gender, the type of disease, the degree of the disease, the other conditions and the like, and is generally preferably in the range of 1×10⁵ to 1×10¹² CFU/g or 1×10⁵ to 1×10¹² CFU/mL, more preferably in the range of 1×10⁶ to 1×10¹² CFU/g or 1×10⁶ to 1×10¹² CFU/mL. When MCC2085 is sterilized cells, CFU/g or CFU/mL can be replaced with cells/g or cells/mL.

The timing of administration of the pharmaceutical composition of the embodiment is not particularly limited, and the timing of administration can be appropriately selected according to the method for treating the subject symptom or disease. The pharmaceutical composition may be administered prophylactically or used for a maintenance therapy. Moreover, the form of administration is preferably determined based on the formulation form, the age of the patient, the gender, the other conditions, the degree of the symptom of the patient or the like. In this regard, in all the cases, the pharmaceutical composition of the embodiment can be administered once a day or in separate portions and may be administered once in several days or in several weeks.

### <Food or Drink Composition>

The food or drink composition of the embodiment is not particularly restricted as long as MCC2085 is contained, and may be a probiotic composition, a nutritional composition, or a general food or drink composition.

The form of the food or drink composition of the embodiment is not particularly limited and may be a powder, granules, a paste, an emulsion, oil drops, capsules, tablets, or the like.

The food or drink composition of the embodiment may be produced by adding MCC2085 to a known food or a known drink, or a new food or drink composition can be produced by mixing MCC2085 into raw material of a food or a drink.

Moreover, the food or drink composition in the embodiment may be a probiotic composition containing a component which has a probiotic effect or a component which supplements a probiotic effect as long as the effects of the invention are not impaired. For example, the food or drink composition in the embodiment can be prepared by combining MCC2085 with a component such as: carbohydrates such as human milk oligosaccharides; proteins such as whey protein, casein protein, soybean protein and pea protein or mixtures or decomposition products thereof; amino acids such as leucine, valine, isoleucine or glutamine; vitamins such as vitamin B6 or vitamin C; creatine; citric acid; or fish oil.

The food or drink composition of the embodiment can be a probiotic composition. The food or drink composition of the embodiment can contain a raw food or drink material in addition to MCC2085. Examples of the raw food or drink material include: saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; and sulfate derivatives such as calcium sulfate.

The food or drink composition of the embodiment can generally contain, per the total dry weight thereof, 1×10³ CFU/g to 1×10¹² CFU/g, more preferably 1×10⁵ CFU/g to 1×10¹¹ CFU/g of MCC2085.

The food or drink composition of the embodiment is not particularly restricted as long as MCC2085 is contained, and examples of the food or drink composition include: drinks such as soft drinks, carbonated drinks, nutritional drinks, fruit juices and lactic acid bacteria beverages (including concentrated undiluted solutions of the drinks and powders for preparation thereof); frozen desserts such as ice creams, sherbets and shaved ice; confectioneries such as sweets, candies, gums, chocolates, tablet candies, snacks, biscuits, jellies, jams, creams and baked sweets; dairy products such as processed milk, milk beverages, fermented milk, drink yogurts and butter; and breads. Moreover, the food or the drink may be a supplement and may be, for example, a supplement in the tablet form. In the case of a supplement, MCC2085 can be taken while the amount of meals and the calorie intake per day are not affected by other foods.

Moreover, the food or drink composition defined in the embodiment can be used as a probiotic composition. That is, the food or drink composition of the embodiment can be provided or sold as a food or a drink with a label with use of probiotics or the like (including a health use). Moreover, the food or the drink can be provided or sold with a label for "those who wish to lead a life with *Bifidobacterium* spp.", "those who want to improve the gut environment", "those who want to regulate the stomach condition", "those who want to form a good gut environment", "those who want to take *Bifidobacterium* spp. which reach the intestines alive", or the like as the subject of intake. The "labeling" act includes all the acts for informing a consumer of the use, and all the expressions which can remind of or cause to guess the use are the "labeling" acts of the invention, regardless of the purposes of labeling, the contents of labeling, the objects to be labeled, the media, and the like.

The "label" is preferably with an expression which allows a consumer to directly recognize the use. Specific examples include an act of transferring an article in which the use is described on a product regarding the food or the drink or packaging of a product, delivering such an article, displaying such an article for transfer or delivery, or importing such an article, and an act of displaying or distributing an advertisement of a product, a price list or a business document with a description of the use thereon or providing information with such contents with a description of the use by an electromagnetic method (specifically the Internet or the like).

The content of the label is preferably a label approved by the administration or the like (for example, a label approved based on various systems provided by the administration and provided in the form based on the approval). It is preferable to label with such a content on packaging, a container, a catalogue, a brochure, an advertisement material in a sales site such as POP (Point of Purchase) advertisement, other documents or the like.

The "labels" also include labels with health foods, functional foods, enteral nutrition products, food for special dietary uses, foods with health claims, foods for specified health uses, foods with nutrient function claims, foods with function claims and the like. In particular, exemplified are labels approved by the Consumer Affairs Agency in Japan, such as labels approved by the systems for foods for specified health uses, foods with nutrient function claims or foods with function claims or a similar system. Specific examples include a label with foods for specified health uses, a label with qualified foods for specified health uses, a label indicating influence on the structure or the function of a body, a label with reduction of disease risk, and a label with a scientifically grounded function. More specifically, typical examples include labels with food for specified health uses (especially labels with health uses) provided by the Cabinet Office Ordinance on Labeling Permission for Special Dietary Uses under the Health Promotion Act (Cabinet Office Ordinance No. 57 on August 31, 2009) and similar labels.

The probiotic composition of the embodiment can be administered to any subject including a non-healthy individual, and is used for the non-therapeutic purpose above when the probiotic composition is a food or a drink labeled with a specific use (especially a health use) or a function.

The food or drink composition according to the embodiment can be used as a nutritional composition. In the embodiment, the "nutritional composition" refers to a food or a drink which is orally taken mainly for the purpose of taking nutrients. The kind of the nutritional composition which can be used in the embodiment is not particularly limited, and is preferably a formula, baby food, liquid food (enteral nutrition products, porridge or the like), a sport drink or the like, more preferably a formula. The subject of intake may be an infant, a small child, a child or an adult, and is preferably an infant or a small child.

The formulas include a powdered formula and a liquid formula. In the present specification, the powdered formula is defined as "those obtained by processing raw milk derived from cow, raw goat's milk, raw sheep's milk, pasteurized goat's milk, cow's milk, certified milk or a food produced therefrom as a raw material, or using such a material as a major raw material, adding nutrients necessary for infants and small children and processing into powder". In the present specification, the liquid formula is defined as "those obtained by processing raw milk derived from cow, raw goat's milk, raw sheep's milk, pasteurized goat's milk, cow's milk, certified milk or a food produced therefrom as a raw material, or using such a material as a major raw material, adding nutrients necessary for infants and small children and processing into liquid".

The formula of the embodiment may further contain a human milk oligosaccharide. The human milk oligosaccharide is not particularly limited as long as it is an oligosaccharide generally contained in human milk, and preferable examples thereof include neutral human milk oligosaccharides such as 2'-fucosyllactose, 3-fucosyllactose, lactodifucotetraose, 2',3-difucosyllactose, lacto-N-triose II, lacto-N-tetraose, lacto-N-neotetraose, lacto-N-fucopentaose I, lacto-N-neofucopentaose, lacto-N-fucopentaose II, lacto-N-fucopentaose III, lacto-N-fucopentaose V, lacto-N-neofucopentaose V, lacto-N-difucohexaose I, lacto-N-difucohexaose II, 6'-galactosyllactose, 3'-galactosyllactose, lacto-N-hexaose and lacto-N-neohexaose, and acidic human milk oligosaccharides such as 3'-sialyllactose, 6'-sialyllactose, 3-fucosyl-3'-sialyllactose and disialyl-lacto-N-tetraose. Of these, 2'-fucosyllactose, 3-fucosyllactose, 3'-sialyllactose and 6'-sialyllactose are preferable, and 2'-fucosyllactose is particularly preferable.

The formulas also include those which contain nutrient components such as various proteins, oils and fats, carbohydrates, minerals and vitamins and which are processed into powder or liquid. As the nutrient component, the nutrient components described above can be used. The formulas further include "a powdered infant formula", "a liquid infant formula" and "a powdered formula for pregnant and parturient women and nursing mothers" of the food for special dietary uses provided by the Health Promotion Act and also include aspects such as a powdered formula for infants and small children for infants of the age of 0 to 12 months, follow-up milk for infants and younger children of the age of 6 to 9 months or older (until the age of 3 years), a powdered formula for low birthweight infants for newborns with a body weight at birth less than 2500 g (low birthweight infants), a powdered formula for an allergy and a powdered formula for lactose intolerance which are used for treating infants and small children with sickness such as milk allergy and lactose intolerance, a powdered formula for inborn errors of metabolism, a powdered formula for small children, nutritional powder for adults, and nutritional powder for the aged.

The formula of the embodiment can be administered to any subject including a healthy individual and a non-healthy individual.

When the formula of the embodiment is administered to a healthy individual, the formula is used for a powdered infant formula, follow-up formula, a powdered formula for low birthweight infants, a powdered formula for small children, nutritional powder for adults, or nutritional powder for the aged, and can be used for the non-therapeutic purpose above.

When the formula of the embodiment is administered to a non-healthy individual, the formula is used for a powdered formula for an allergy, a powdered formula for lactose intolerance or a powdered formula for inborn errors of metabolism, and can be used for the therapeutic purpose above.

The nutritional composition according to the embodiment can be applied to food with health claims or food for the ill. The system for food with health claims has been established not only for general foods but also for foods in the form of tablets, capsules or the like, in view of the trends inside and outside of Japan, and also considering the consistency with the existing system for foods for specified health uses. The system includes two types, namely foods for specified health uses which are of the individual approval system and foods with nutrient function claims which are of the standard regulation system.

### <Production Method of Composition>

The method for producing a composition of the embodiment is explained below. In the method for producing a composition of the embodiment, the step of adding MCC2085 may be conducted at any stage in the production step of the composition.

The method for producing a composition includes at least a step of mixing MCC2085 and raw material. As MCC2085, one described above can be used. Examples of the raw material include the raw material of the food or drink composition described above, a component which has a probiotic effect, a component which supplements a probiotic effect, and a carrier for formulation. In the case of a method for producing a composition for a food or a drink, at least the raw material of the food or drink composition described above is contained as the raw material. In the case of a method for producing a composition for a food or a drink, at least one kind of a component which has a probiotic effect, a component which supplements a probiotic effect, a carrier for formulation and the like may be contained as the raw material.

When MCC2085 and the raw material are mixed, MCC2085 and the raw material are preferably mixed in such a manner that the MCC2085 content based on the total mass of the composition becomes 1×10³ to 1×10¹² CFU/g, further preferably 1×10⁵ to 1×10¹¹ CFU/g.

The method for producing a composition may further include a step of drying a mixture of MCC2085 and the raw material. The drying method is not particularly limited as long as the effects of the embodiment are not impaired. A specific drying method may be a spray drying method or a lyophilization method.

The method for producing a composition may further include a step of sterilizing the mixture of MCC2085 and the raw material. The sterilization method is not particularly limited as long as the effects of the embodiment are not impaired, but examples thereof include a retort sterilization method, a lyophilization method, a UHT sterilization method, a pressurized sterilization method, a high-pressure steam sterilization method, a dry heat sterilization method, a flow steam sterilization method, an electromagnetic wave sterilization method, an electron beam sterilization method, a high-frequency sterilization method, a radiation sterilization method, a ultraviolet sterilization method, an ethylene oxide gas sterilization method, a hydrogen peroxide gas plasma sterilization method, and a chemical sterilization method (specifically, an alcohol sterilization method, a formalin fixation method, and an electrolyzed water treatment method).

The method for producing a composition may include at least one of a step of further mixing a prebiotic in the mixture and a step of further mixing a milk component in MCC2085 or the mixture.

The method for producing a composition may further include a step of mixing at least one kind of bacterium of probiotics such as *Bifidobacterium* strains other than MCC2085 and lactic acid bacteria.

The milk component is preferably a powder and is more preferably mixed with a bacterial powder of MCC2085.

The milk component is not particularly limited as long as the effects of the embodiment are not impaired. Examples of the milk component include cow's milk, buffalo's milk, sheep's milk, goat's milk, mare milk, skim milk, concentrated skim milk, skim milk powder, concentrated milk, whole milk powder, cream, butter, buttermilk, condensed milk, and a milk protein. One kind or two or more kinds selected from the group consisting thereof can be used. The milk protein is not particularly limited, and examples thereof include whey, casein, and hydrolysates thereof. One kind or two or more kinds selected from the group consisting thereof can be used. The milk component is preferably a component derived from cow's milk.

Regarding the hydrolysates, a whey hydrolysate, a casein hydrolysate or the like can be produced by hydrolyzing the milk component (preferably a milk protein). Examples of the hydrolysis include acid or alkali hydrolysis and enzymatic hydrolysis. Of these, enzymatic hydrolysis is preferable. The enzyme used for enzymatic hydrolysis is preferably a proteolytic enzyme. Examples of the proteolytic enzyme include protease, trypsin, chymotrypsin, plasmin, pepsin, papain, peptidase, and aminopeptidase. The pH during the hydrolysis reaction is appropriately adjusted to the optimum pH of the enzyme used, and the hydrolysis reaction can be conducted, for example, at pH 2 to 6. The temperature during the hydrolysis reaction is not particularly limited and is generally preferably in the range of 30 to 60°C. The reaction period is preferably two to 10 hours.

When a supplement or tablets are produced as the composition of the invention, the method for producing a composition of the embodiment can include a step of mixing a culture containing MCC2085 and a carrier for formulation or the like and thus obtaining a mixture and a step of forming the mixture into the predetermined shape of the supplement or the tablets. The forming step is, for example, tableting. An example of tableting is obtaining tablets by compression molding of a powder or a granulated mixture.

Furthermore, an example of the method for producing a composition of the embodiment is a method for producing a fermented food or drink (preferably a fermented milk) containing MCC2085. A step of adding a dried mixture of MCC2085 and the raw material to a raw fermented milk material and thus obtaining a fermented milk containing MCC2085 is conducted. Alternatively, a step of mixing a dried mixture of MCC2085 and the raw material and a fermented milk and thus obtaining a fermented milk containing MCC2085 is conducted. The fermentation condition and the blending amount are adjusted in such a manner that the bacterial cell amount of MCC2085 in the composition becomes the bacterial cell amount described above. As a result, a fermented food or drink, preferably a fermented milk or a fermented product, containing MCC2085 can be provided.

### <Method for Improving Intestinal Microbiota and Use of Bacterium for Improvement of Intestinal Microbiota>

The method for improving intestinal microbiota of the invention includes administering a bacterium which is *Bifidobacterium bifidum* MCC2085 (NITE BP-03881) to a subject of administration at an intake of 1×10³ to 1×10¹² CFU/day.

In the method for improving intestinal microbiota of the embodiment, MCC2085 can be administered to any subject including the healthy individual and the non-healthy individual above. When MCC2085 is administered to a subject of administration who feels a problem with the intestines, such as constipation and diarrhea, at an intake of 1×10³ to 1×10¹² CFU/day, the intestinal microbiota can be expected to be improved. The intake of MCC2085 by the subject of administration is preferably conducted continuously.

That is, the bacterium which is *Bifidobacterium bifidum* MCC2085 (NITE BP-03881) can be used for improving the intestinal microbiota of the subject of administration.

According to another aspect of the invention, an oral composition for reaching an intestine containing *Bifidobacterium bifidum* MCC2085 (NITE BP-03881) can be provided.

MCC2085 has high acid resistance and thus can maintain the survival rate high even with coexistence with gastric acid when MCC2085 is orally taken. As a result, when MCC2085 is orally taken, MCC2085 can reach the intestines while having the high survival rate.

The matters regarding MCC2085 can all be in accordance with the above contents.

### Examples

The embodiment is explained in further detail below based on Examples. In this regard, the Examples explained below show examples of typical Examples of the embodiment, and the scope of the invention is not construed as being narrower due to the Examples.

### [Test Example 1]

### (1) Object

Test Example 1 was conducted to acquire *Bifidobacterium bifidum* MCC2085 and to elucidate the bacteriological properties of the bacterium.

### (2) Acquisition and Bacteriological Properties of Bifidobacterium bifidum MCC2085

MCC2085 was isolated from feces of an infant. MCC2085 was anaerobically cultured on a BL agar plate (Eiken Chemical Co., Ltd., E-MG16, containing 5% sterile defibrinated horse blood) at 37°C for 48 hours, and the formed colonies were observed. The bacteriological properties are shown in Table 1.

**[Table 1]**

| | Properties |
|---|---|
| Diameter | 2 to 3 mm |
| Color | Brown |
| Shape | Circle |
| Protrusion State | Flat |
| Circumference | Entire |
| Surface Shape and the like | Smooth |
| Transparency | Opaque |
| Consistency | Butter-like |

### (3) Analysis of 16S rRNA Gene Base Sequence

The base sequence of a 16S ribosomal RNA (16S rRNA) gene of MCC2085 was analyzed. Based on the MCC2085 cultured in (2) above, a sample DNA solution used for PCR was prepared, and the 16S rRNA gene was amplified by PCR using the sample DNA solution to obtain an amplified DNA product. The 16S rRNA gene sequence of MCC2085 was determined therefrom.

As a result of homology search for the full length 16S rRNA gene sequence on the international base sequence database (Genbank) of the NCBI (National Center for Biotechnology Information) with BLAST (Basic Local Alignment Search Tool) based on the base sequence information, 100% homology with *Bifidobacterium bifidum* ATCC29521 was shown.

The term "100% homology with *Bifidobacterium bifidum* ATCC29521" should be understood that both MCC2085 (accession number NITE BP-03881) and ATCC29521 share an evolutionary relationship of 100% in the 16S rRNA gene sequences thereof.

Thus, as the present inventors have observed that both strains have 100% sequence identity in 16S rRNA, both strains share a common evolutionary ancestor. As a result, it was shown that novel MCC2085 deposited with the name *Bifidobacterium bifidum* MCC2085 (accession number NITE BP-03881) in fact belongs to species *Bifidobacterium bifidum* and is evolutionary related to ATCC29521.

Furthermore, average nucleotide identity (ANI) analysis of the whole genome sequences of ATCC29521 and MCC2085 was conducted using ANI blast of pyANI (v0.2.12). As a result, the ANI value of both was 99.00%, and it was elucidated that ATCC29521 and MCC2085 are different strains.

As shown in Test Example 2 and Test Example 3 below, it was shown that MCC2085 has surprisingly superior acid resistance to that of ATCC29521 and that the serotonin production amount of MCC2085 is significantly high. Accordingly, it is obvious that *Bifidobacterium bifidum* MCC2085 (NITE BP-03881) described in the present specification belongs to species *Bifidobacterium bifidum* but is a different strain from ATCC29521.

### [Test Example 2]

### (1) Object

Test Example 2 was conducted to evaluate the acid resistance of *Bifidobacterium bifidum* MCC2085.

### (2) Bacteria

The two types of bacteria below were used.
- *Bifidobacterium bifidum* MCC2085 (NITE BP-03881)
- *Bifidobacterium bifidum* (ATCC29521, Type stain)

ATCC29521 can be acquired from the American Type Culture Collection. ATCC is located at 10801 University Boulevard, Manassas, VA 20110, United States of America.

### (3) Preparation of Artificial Gastric Juice

Sodium chloride at a final concentration of 0.2 mass% and pepsin (manufactured by FUJIFILM Wako Pure Chemical Corporation) at a final concentration of 0.35 mass% were added to ultrapure water and mixed with a stirrer. The obtained mixture was adjusted to pH 3.0 using hydrochloric acid and then filtered using a filter having 0.45um pores, and thus artificial gastric juice was obtained.

### (4) Preculture of Bacteria

The two types of bacteria were anaerobically cultured in an MRS culture medium containing 0.05 mass% cysteine hydrochloride at 37°C for 16 hours.

### (5) Acid Resistance Test

The two types of bacteria were each cultured in the presence of the artificial gastric juice for 0 minute, 30 minutes and 120 minutes, and the bacterial counts were counted over time.

### (5-1) Culture Period of 0 Minute

The bacteria after the preculture were diluted 10-fold using saline solution and then again diluted 100-fold twice with saline solution, and thus 10⁵-fold diluted bacterial cell-containing solutions were prepared. The obtained 10⁵-fold diluted bacterial cell-containing solutions were diluted 10-fold twice, and thus 10⁶-fold diluted bacterial cell-containing solutions and 10⁷-fold diluted bacterial cell-containing solutions were obtained. The three kinds of diluted bacterial cell-containing solutions were used as samples with reaction period with the artificial gastric juice of 0 minute.

### (5-2) Culture Periods of 30 Minutes and 120 Minutes

The bacteria after the preculture were diluted 10-fold using saline solution, then diluted 10-fold with the artificial gastric juice and reacted at 37°C using a heat block for 30 minutes or 120 minutes. After the completion of the reaction, the solutions were diluted 10-fold using Mitsuoka's buffer and further diluted 10-fold four times with saline solution, and thus 10⁵-fold diluted, 10⁶-fold diluted and 10⁷-fold diluted bacterial cell-containing solutions were prepared. Mitsuoka's buffer contains 4.5 g of potassium dihydrogen phosphate, 6.0 g of disodium hydrogen phosphate, 0.5 g of L-cysteine hydrochloride, 0.5 g of Tween (registered trademark) 80 and 1.0 g of agar in 1 L of ultrapure water.

### (5-3) Measurement of Viable Bacterial Counts

The three types of diluted bacterial cell-containing solutions prepared with the respective culture periods (0 minute, 30 minutes and 120 minutes) in an amount of 100 µL (/dish) were added to a TOS culture medium (Yakult Pharmaceutical Industry Co., Ltd.) and pour-cultured under an anaerobic condition. After the pour culture, the bacteria were cultured at 37°C for 72 hours. After the culture, the colonies were counted, and the bacterial cell concentrations (CFU/ml) were calculated.

The bacterial cell concentrations were calculated by conducting the procedures (5-1) to (5-3) three times each for the bacterial cell dilutions (the 10⁵-, 10⁶- and 10⁷-fold diluted bacterial cell-containing solutions) with the respective culture periods (0, 30 and 120 minutes) and determining the arithmetic average values (n=3).

The survival rates of the bacteria after the respective culture periods were calculated with the following equation.

Survival rate (%) = bacterial cell concentration (CFU/ml) with arbitrary culture period/bacterial cell concentration (CFU/ml) with culture period of 0 minute × 100

### (6) Results

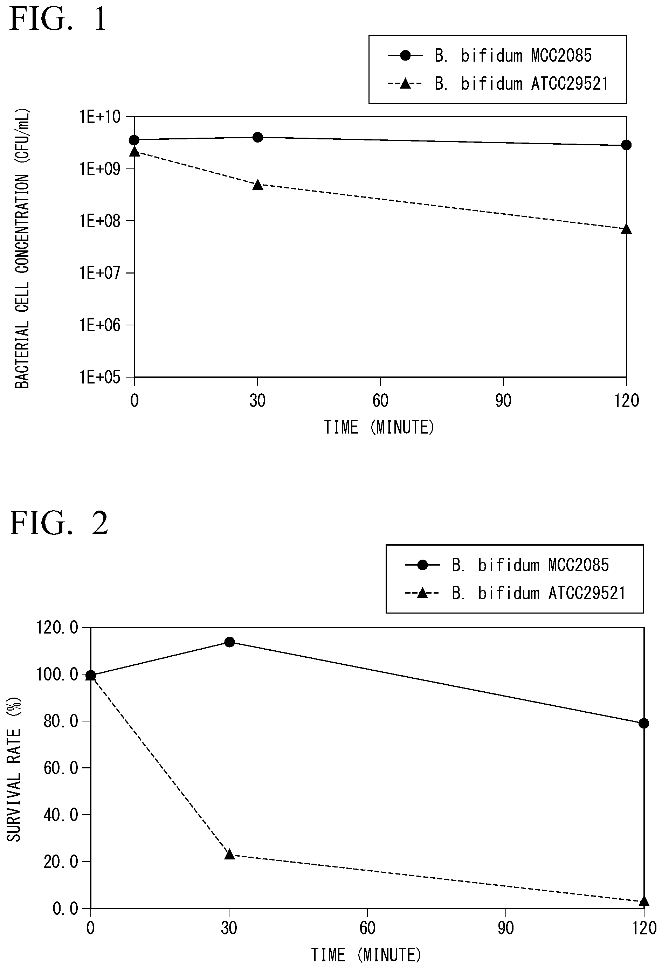

The results of the changes in the bacterial cell concentrations with time are shown in FIG. 1. The data of MCC2085 are those of the 10⁷-fold diluted bacterial cell-containing solution, and the data of ATCC29521 are those of the 10⁶ diluted bacterial cell-containing solution. As shown in FIG. 1, the bacterial cell concentration of MCC2085 hardly changed even after the culture period of 120 minutes compared to the bacterial cell concentration after culturing for 0 hour and remained at a bacterial cell concentration of around 10⁹ CFU/ml. On the other hand, the bacterial cell concentration of ATCC29521 was around 10⁹ CFU/ml after culturing for 0 minute, but decreased to around 10⁷ CFU/ml after the culture period of 120 minutes.

The results of the changes in the survival rates of the bacterial cells with time are shown in FIG. 2.

As shown in FIG. 2, the survival rate of MCC2085 after culturing for 120 minutes was 79%. On the other hand, the survival rate of ATCC29521 was 23% after culturing for 30 minutes and 3.1% after culturing for 120 minutes.

Thus, it was found that MCC2085 has high acid resistance to artificial gastric juice.

### [Test Example 3]

### (1) Object

Test Example 3 was conducted to quantify the serotonin production amounts by *Bifidobacterium bifidum* MCC2085 (NITE BP-03881) and *Bifidobacterium bifidum* ATCC29521.

### (2) Preparation of Bacterial Cell-Containing Culture Solutions

MRS culture medium (Difco^{™} Lactobacill MRS Broth, type number 288130) was sterilized at 121°C for 15 minutes. MCC2085 or ATCC29521 was cultured in the MRS culture medium after sterilization under an anaerobic condition at 37°C for 16 hours. After the culture solutions after the culture were centrifuged to collect the bacterial cells, the collected bacterial cells were suspended in phosphate buffered saline (PBS) and adjusted to 1×10⁹ cells/ml, and thus solutions containing MCC2085 or ATCC29521 were obtained.

### (3) Culture and Seeding of RIN14B Cells

RIN14B cells (ATCC CRL-2059, rat pancreatic cancer-derived enterochromaffin-like cells) were cultured in an RPMI 1640 culture medium containing 10% fetal bovine serum (FBS) and 1% penicillin-streptomycin (manufactured by FUJIFILM Wako Pure Chemical Corporation) under a condition at 37°C and 5% CO₂ and subcultured once in three to four days.

The RIN14B cells after the culture were incubated using 0.25 w/v% trypsin/EDTA (gibco) at 37°C for five minutes and diluted using the RPMI 1640 culture medium containing 10% FBS and 1% penicillin-streptomycin, and thus a solution containing 2.5×10⁵ cells/mL of RIN14B cells was obtained. The RIN14B cell-containing solution was seeded in a 24-well plate at 500 µL/one well, and then the cells were further cultured under a condition at 37°C and 5% CO₂ for 48 hours.

### (4) Quantification of Serotonin Production Amount

The RIN14B cells after culturing in (3) above were washed once with an HBSS(+) solution (FUJIFILM Wako Pure Chemical Corporation). The MCC2085- or ATCC29521-containing solutions prepared in (2) above were diluted 20-fold using an RPMI 1640 culture medium containing 1% penicillin-streptomycin and added to the wells at 500 µL/one well, and the cells were cultured under a condition at 37°C and 5% CO₂ for 24 hours. After culturing for 24 hours, the culture supernatants were collected, and the serotonin contents of the culture supernatants were measured using an ELISA kit (manufactured by DLD, type number EA602/96).

Here, the same procedures as those above were conducted except that the bacterial cell-containing solutions were not added to obtain a control.

### (5) Results

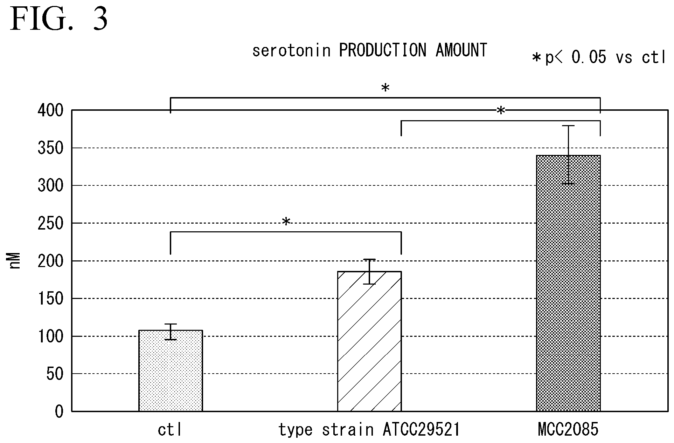

The serotonin production amounts of MCC2085 and ATCC29521 are shown in FIG. 3. For the statistical analysis, the groups were compared by t-test, and correction with the p value was conducted using bonferroni.

The serotonin production amounts of MCC2085 and ATCC29521 increased with significance compared to that of the control without the addition. The serotonin production amount of MCC2085 increased with significance compared to that of ATCC29521. The ratios of the serotonin production amounts of the bacteria MCC2085 and ATCC29521 to that of the control were 3.2 and 1.7, respectively.

Other bacterial strains were also tested (data not shown). The serotonin production effects of the bacterial strains were measured using an ELISA kit (manufactured by DLD, type number EA602/96). The measured serotonin production amounts in the supernatants were equivalent to the production amount of ATCC29521. However, *Bifidobacterium bifidum* MCC2085 (NITE BP-03881) was superior to these test strains.

### [Production Examples]

Production Examples of the composition, the pharmaceutical composition, the fermented milk, the powdered formula and the food or drink composition of the embodiment are shown below, but the composition of the embodiment is not limited thereto.

### [Production Example 1]

MCC2085 is added to 3 mL of an MRS liquid culture medium (Difco^{™} Lactobacill MRS Broth, type number 288130) and anaerobically cultured at 32 to 39°C for five to 25 hours, and then the culture solution is concentrated and lyophilized to obtain lyophilized powder of the bacterium (namely bacterial powder). The bacterial powder and whey protein concentrate (WPC) are mixed uniformly, and thus a composition is obtained. The composition in an amount of 20 g is dissolved in 200 g of water, and thus a composition containing MCC2085 is obtained.

### [Production Example 2]

MCC2085 is added to 3 mL of an MRS liquid culture medium and anaerobically cultured at 32 to 39°C for five to 25 hours, and then the culture solution is concentrated and lyophilized to obtain lyophilized powder of the bacterium (namely bacterial powder). The bacterial powder and dry powder of milk protein concentrate (MPC480, manufactured by Fonterra Co-operative Group Limited, protein content of 80 mass%, casein protein:whey protein = about 8:2) are mixed uniformly, and thus a composition is obtained. The composition in an amount of 20 g is dissolved in 200 g of water, and thus a composition containing MCC2085 is obtained.

### [Production Example 3]

MCC2085 is added to 3 mL of an MRS liquid culture medium and anaerobically cultured at 32 to 39°C for five to 25 hours, and then the culture solution is concentrated and lyophilized to obtain lyophilized powder of the bacterium containing *Bifidobacterium bifidum* MCC2085 (NITE BP-03881) (namely bacterial powder). Next, the bacterial powder and crystalline cellulose are introduced into an agitation granulator and mixed. Then, purified water is added for granulation, and the granules are dried to obtain granules containing an excipient (a pharmaceutical composition). In this manner, granules containing MCC2085 can be obtained.

### [Production Example 4]

A production method of a fermented milk containing MCC2085 is shown below.

First, a raw milk material, water according to the need, other components and the like are mixed, preferably homogenized and heat sterilized. The homogenization and the heat sterilization can be conducted by general methods. A lactic acid bacterium starter is added (namely seeded) to the heat sterilized prepared milk solution, and the solution is kept at a predetermined fermentation temperature and fermented to obtain a fermented product. Through fermentation, curd is formed.

As the lactic acid bacterium starter, for example, lactic acid bacteria which are generally used for yogurt production, such as *Lactobacillus bulgaricus, Lactococcus lactis* and *Streptococcus thermophilus,* can be used. When the pH reaches the target value, the formed curd is crushed by stirring and cooled to 10°C or lower, and a fermented product is thus obtained. By cooling to 10°C or lower, the activity of the lactic acid bacterium can be reduced, and the production of an acid can be suppressed.

Next, the fermented product obtained by the fermentation step is heat treated, and a fermented product after the heating is thus obtained. By appropriately heating the fermented product, the production of an acid by the lactic acid bacterium in the fermented product after the heating can be inhibited. Thus, a decrease in pH during the subsequent production step and/or during the storage of the concentrated fermented milk containing the strain of *Bifidobacterium* can be inhibited. As a result, the viability of the strain of *Bifidobacterium* can be improved.

Next, MCC2085 is added to the fermented product after the heating obtained by the heat treatment step. The amount of MCC2085 added, relative to the fermented product after the heating, is preferably 1×10⁷ to 1×10¹¹ CFU/mL, more preferably 1×10⁸ to 1×10¹⁰ CFU/mL. When MCC2085 is sterilized cells, the CFU/mL can be replaced with the cells/mL.

After adding MCC2085 to the fermented product after the heating, the mixture is concentrated. The concentration step can be conducted appropriately using a known concentration method. For example, centrifugation method or membrane separation method can be used. Through the centrifugation method, the whey in the product to be concentrated (namely, the fermented product after the heating to which MCC2085 has been added) is removed, and a concentrated fermented milk which has an increased solid content concentration is obtained.

A fermented milk containing the obtained MCC2085 can be produced as described above.

### [Production Example 5]

A production method of a powdered formula containing MCC2085 is shown below.

In 300 kg of warm water, 10 kg of desalted cow's milk whey protein powder (manufactured by MILEI GmbH), 6 kg of cow's milk casein powder (manufactured by Fonterra Cooperative Group Limited), 48 kg of lactose (manufactured by MILEI GmbH), 920 g of a mineral mixture (manufactured by Tomita Pharmaceutical Co., Ltd), 32 g of a vitamin mixture (manufactured by Tanabe Seiyaku Co., Ltd.), 500 g of lactulose (manufactured by Morinaga Milk Industry Co., Ltd.), 500 g of raffinose (manufactured by Nippon Beet Sugar Manufacturing Co., Ltd.) and 900 g of galactooligosaccharide syrup (manufactured by Yakult Pharmaceutical Industry Co., Ltd.) were dissolved and further heated and dissolved at 90°C for 10 minutes, and after adding 28 kg of modified fats (manufactured by Taiyo Yushi Corp.), the mixture was homogenized. Then, after sterilization and concentration steps, the mixture was spray dried, and about 95 kg of a powdered formula was thus prepared. To the powdered formula, 100 g of bacterial powder of MCC2085 (10⁸ to 10¹¹ CFU/g, manufactured by Morinaga Milk Industry Co., Ltd.) triturated in starch is added, and about 95 kg of a powdered formula containing the strain of *Bifidobacterium* and oligosaccharides is thus prepared. When the obtained powdered formula is dissolved in water and a formula solution having a total solid content concentration as a standard formula concentration of 14% (w/V) is obtained, 10⁶ to 10⁹ CFU/100 mL can be obtained as the *Bifidobacterium* strain count in the formula solution.

By taking or administering the powdered formula containing MCC2085 obtained in the above manner, the enteric environment of a human can be improved.

### [Production Example 6]

A production method of a food for improving enteric environment containing MCC2085 is shown below.

MCC2085 is added to 3 mL of an MRS liquid culture medium and anaerobically cultured at 32 to 39°C for five to 25 hours, and then the culture solution is concentrated and lyophilized to obtain lyophilized powder of the bacterium (namely bacterial powder) and to thus obtain a composition of the embodiment. The bacterial powder of the embodiment can be added to a food containing a large amount of cellulose (for example, vegetables or salad) and cooked, and a composition of the embodiment can also be thus obtained.

Alternatively, the bacterial powder is taken at a meal, before a meal, during a meal or after a meal. Because dietary fibers are automatically taken with a regular diet (a diet with staple food, a side dish and a main dish), an effect of improving the enteric environment of an adult can be expected when the dry bacterial powder product of the embodiment is taken together during a meal.

When the food for improving enteric environment of the embodiment is continuously taken by a person feeling a problem with the intestines, such as constipation and diarrhea, a higher effect of improving enteric environment can be expected.

Therefore, *Bifidobacterium bifidum* MCC2085 (NITE BP-03881) and a composition containing *Bifidobacterium bifidum* MCC2085 (NITE BP-03881) can help proliferation of *Bifidobacterium* spp. in the intestines and can be used effectively for formation of excellent intestinal microbiota, improvement of enteric environment and the like.

## Claims

1. A bacterium which is *Bifidobacterium bifidum* MCC2085 (NITE BP-03881).

2. A composition comprising the bacterium according to claim 1.

3. The composition according to claim 2 which is a food or drink composition.

4. The composition according to claim 3, wherein the food or drink composition is a probiotic composition or a nutritional composition.

5. The composition according to claim 4, wherein the nutritional composition is a formula.

6. The composition according to claim 4 or 5, further comprising a human milk oligosaccharide.

7. The composition according to claim 2 which is a pharmaceutical composition.

8. A method for producing a composition comprising a step of mixing the bacterium according to claim 1 and raw material.
